(19) Europäisches Patentamt
European Patent Office
Office européen des brevets

(11) **EP 3 713 435 B1**

(12) **EUROPEAN PATENT SPECIFICATION**

(45) Date of publication and mention
of the grant of the patent:
**24.01.2024 Bulletin 2024/04**

(21) Application number: **18833393.4**

(22) Date of filing: **23.11.2018**

(51) International Patent Classification (IPC):
**A41D 13/00** *(2006.01)*      **A61F 13/08** *(2006.01)*

(52) Cooperative Patent Classification (CPC):
**A61F 13/085**

(86) International application number:
**PCT/EP2018/082453**

(87) International publication number:
**WO 2019/101972 (31.05.2019 Gazette 2019/22)**

(54) **MODULAR GARMENT SYSTEM AND USE THEREOF**

MODULARES BEKLEIDUNGSSYSTEM UND VERWENDUNG DAVON

SYSTÈME DE VÊTEMENT MODULAIRE ET SON UTILISATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **24.11.2017 EP 17203619**

(43) Date of publication of application:
**30.09.2020 Bulletin 2020/40**

(73) Proprietor: **Adornato, Sandro
Bitti NU (IT)**

(72) Inventor: **Adornato, Sandro
Bitti NU (IT)**

(74) Representative: **CH Kilger Anwaltspartnerschaft
mbB
Fasanenstraße 29
10719 Berlin (DE)**

(56) References cited:
**CA-A1- 2 297 593        US-A- 4 215 687
US-A- 4 961 418        US-A1- 2007 179 421
US-A1- 2011 009 793**

**Description**

**FIELD OF THE INVENTION**

The present invention is in the field of medical and sport devices, particularly in the field of garment devices. More specifically, the present invention relates to a modular garment system. The invention is also in the field of physical medicine and rehabilitation and relates to the use of a modular garment system for rehabilitation and training of a subject in need thereof.

**BACKGROUND**

In the last decade, compression garments gained new appeal by expanding their applications beyond the original medical use such as in sport settings for improving performance of athletes or in injury prevention and rehabilitation.

The principle of compression garments originates from compression therapy for treating subjects with lower limb diseases concerning venous, lymphatic and arterial vessels. Such therapy comprises applying a known amount of pressure to promote return of venous blood to the heart and provide support to the circulatory system.

With reference to fitness applications, the benefit of using compression garments has been investigated in studies analyzing several parameters (e.g., maximal strength, sprint performance, jump height, time to exhaustion, body temperature, muscle swelling, blood lactate removal, heart rate response) in different subjects and sport disciplines. However, the effects of wearing those compression garments on performance and recovery is still matter of debate due to the heterogeneous results of medical studies. Nevertheless, it appears to be commonly accepted that compression garments can improve performance and motor skill precision. Neuromuscular benefits from using compression garments include reduced muscle oscillation, improved joint awareness, augmented perfusion, higher skin temperature and improved oxygen utilization (Born et al. 2013, Int. J. Sports Physiol. Perform. 8(1):4-18).

Compression clothing are provided in different forms based on the subject's needs and/or sport discipline. In running and cycling, for instance, lower body compression clothing such as knee-high socks, shorts, and full-length tights are the most common types of compression garments (Bringard et al. 2006, Int. J. Sports Med. 27(5):373-378; Scanlan et al. 2008, Int. J. Sports Physiol. Perform. 3(4):424-438).

Compression garments are also available in different degrees of compression depending on the body area intended to cover and the purposed effect aimed to obtain. Accordingly, a lot of efforts have been made in terms of engineering to develop new materials and design capable to satisfy more demanding needs.

For example, the U.S. patent No. 4,215,687 describes a body or limb encircling therapeutic device which applies a compressive force on or support to the body or limb. The device comprises a plurality of body or limb encircling bands, each individually tightened to apply the desired pressure to the body or limb, and an anchoring tape having an interlocking fabric material on one side maintaining adjacent bands of the therapeutic device in their proper edge-to-edge relationship.

The U.S. patent application No. US2011/0009793A1 describes an adjustable compression garment comprising a first fabric portion that is moveable relative to a second fabric portion and positioned on a torso portion or a limb portion of the garment. A fastening arrangement includes a first fastener device connected to the first fabric portion and a second fastening device connected to the second fabric portion. The fastening device is configured such that the degree of compression provided by the first fabric portion may be increased without disconnecting the first fastener device from the second fastener device.

A modular compression garments comprising a plurality of bands, wherein each band is designed to provide a predetermined level of compression at maximal stretch is also described in the U.S. patent application No. US2007/0179421A1.

The patent application in Canada No. 2,297,593 describes a compression garment system comprising a short which applies compression in the desired areas while resisting dislodgement during movement and enhancing heat retention in the desired area and a hip spice to supplement the compression effect of the short.

An example of new materials for compression garments is disclosed in the U.S. patent No. 4,961,418, which describes a composite fabric structure and its use in garments that provide improved heat, compression and skin stimulation therapy.

An important requirement that every compression garment should possess is its capability to fit with a subject's body and provide an appropriate degree of compression. In this regard, if compression is not optimized, then the garment is not capable of doing what it is proposed to do, regardless of whether that compression can make any physiological difference to the subject wearing such garment.

Not all the compression garments meet such requirement. This is also partly confirmed by the fact that most of studies do not report the value of compression the subjects wearing such garments receive and thus, contributing to the large variability of the results obtained in this matter.

Finally, most of the commercially available compression garments are not suitable for different types of physical activities since in some cases they may limit the subject's movement or lose their original position due to a prolonged workout activity and become uncomfortable.

Therefore, it is desirable to provide an improved compression garment system that would satisfy the aforementioned requirements and overcome the drawbacks of current compression garment products.

## SUMMARY OF THE INVENTION

A first aspect of the present invention relates to a modular garment system, the system comprising:

    a. a jacket-like module, and
    b. a trouser-like module,

wherein each of said modules comprises at least two fastening elements configured to provide a resistance and/or a support to the movement of a subject's body being contacted by said at least two fastening elements and a compression to said subject's body being contacted by said at least two fastening elements,
wherein said at least two fastening elements are made by an elastic fabric selected from the group comprising elastic Velcro, Elastam or a combination thereof,
wherein said at least two fastening elements comprise

    a. at least one fastening element of first type (1), and
    b. at least one fastening element of second type (2),

wherein said at least one fastening element of first type (1) is arranged in an overlapping region between two adjacent modules and in an overlapping region within the same module, and wherein said at least one fastening element of second type (2) is arranged in a region of the module contacting a skeletal muscle of said subject's body.

A second aspect of the present invention relates to a kit for rehabilitation and/or training of a subject, the kit comprising:

    a. a modular garment system according to the first aspect,
    b. an elastic band (3), and
    c. optionally, a hood-like module,

wherein the elastic band (3) is attached to the modular garment system by means of fastening elements of second type (2) arranged on said band (3) that contact corresponding fastening elements of first (1) and second (2) type arranged on the jacket-like module and trouser-like module.

## BRIEF DESCRIPTION OF THE DRAWINGS

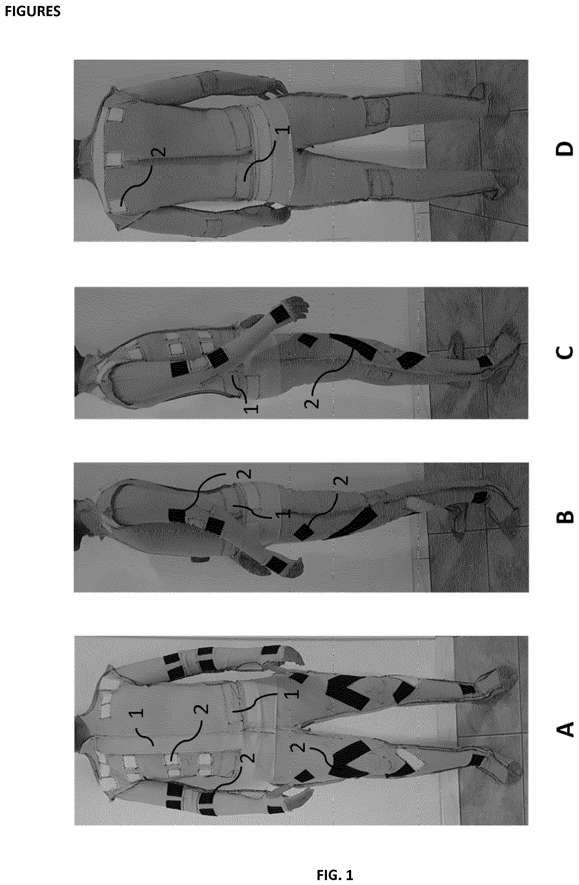

Figure 1 shows front (A), lateral (B, C) and back (D) view of the modular garment system according to the present invention. Figure 1 also shows positions of fastening elements of first (1) and second (2) type characterizing the jacket-like module and the trouser-like module.

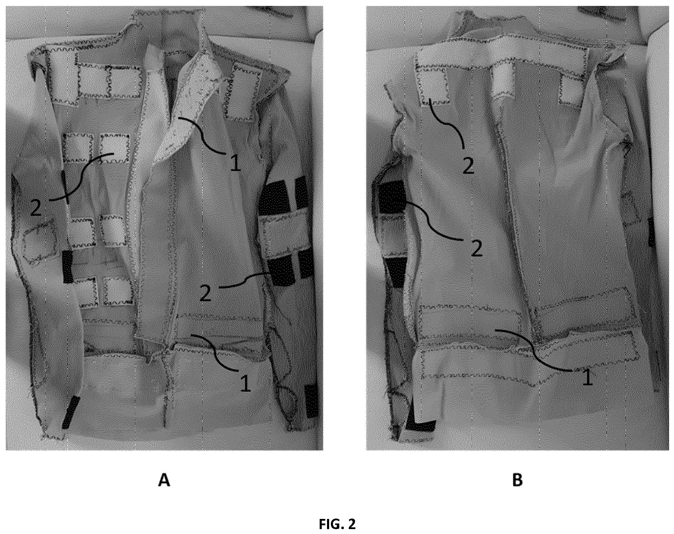

Figure 2 shows front (A) and back (B) view of the jacket-like module comprising fastening elements of first (1) and second (2) type.

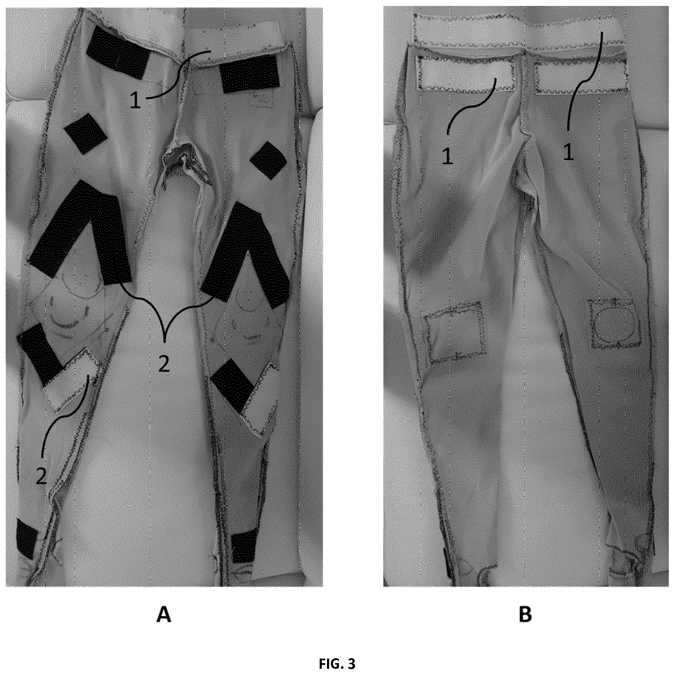

Figure 3 shows front (A) and back (B) view of the trouser-like module comprising fastening elements of first (1) and second (2) type.

Figure 4 shows a non-limiting example of the elastic band (3) mimicking static posterior chain. The elastic band (3) can be integrated in the modular garment system by means of fastening elements of second type (2) arranged on said band (3) that contact corresponding fastening elements of first (1) and second (2) type arranged on the jacket-like module and/or trouser-like module. The elastic band (3) can have different sizes and shapes according to the body's area of a subject intended to cover.

Figure 5 shows the measurement of longitudinal (A) and transversal (B) force wherein the fabric (without fastening elements of first (1) and second (2) type) is subjected to stretch. Also, Figure 5 shows the transversal reaction force of the fabric (without fastening elements of first (1) and second (2) type) in reply to a longitudinal stretch (C).

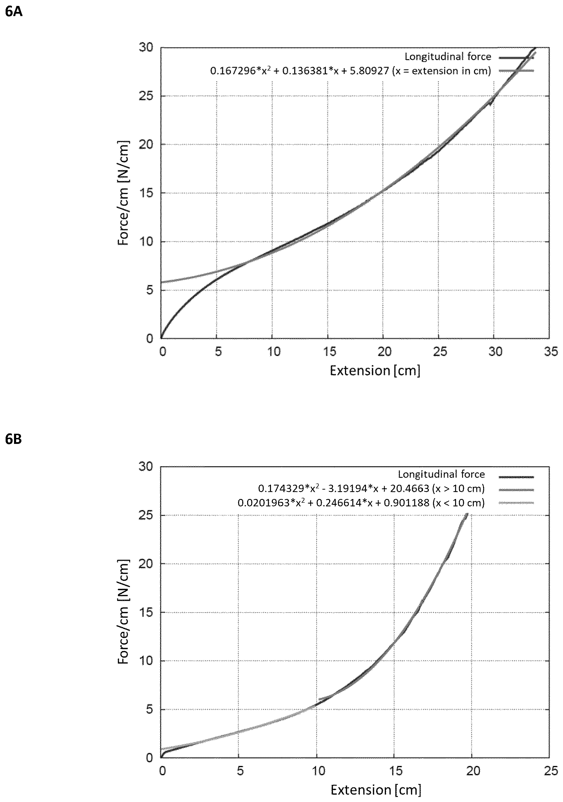

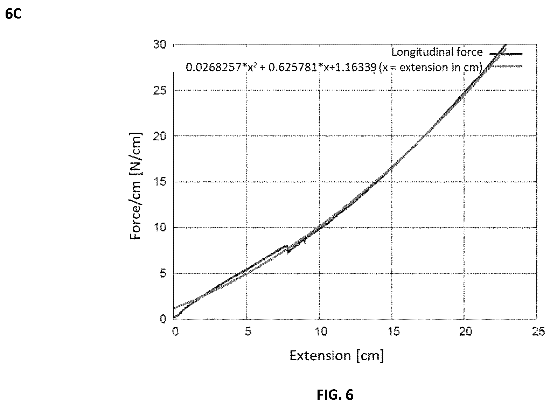

Figure 6(A) shows the measurement of longitudinal forces exerted by the fabric and/or the elastic band (3) composed by 30% Velcro and 70% Elastam (measuring 20*10cm) with two fastening elements (measuring 5*7cm each) positioned longitudinally. Figure 6(B) shows the measurement of longitudinal forces exerted by only one elastic band composed only by Velcro (measuring 20*5cm).Figure 6(C) shows the measurement of longitudinal forces exerted by the fabric and/or only one elastic band composed only by Velcro 30% transversely positioned and Elastam 70%. Said fastening elements are encompassed by the modular garment system according to the present invention.

Figure 7 shows the fabric response to stress. In particular, the tested fabric sample (size, 20 * 10cm) has been stressed up to 100 N (Figure 7A) and up to 300 N (Figure 7B), both for 5 cycles.

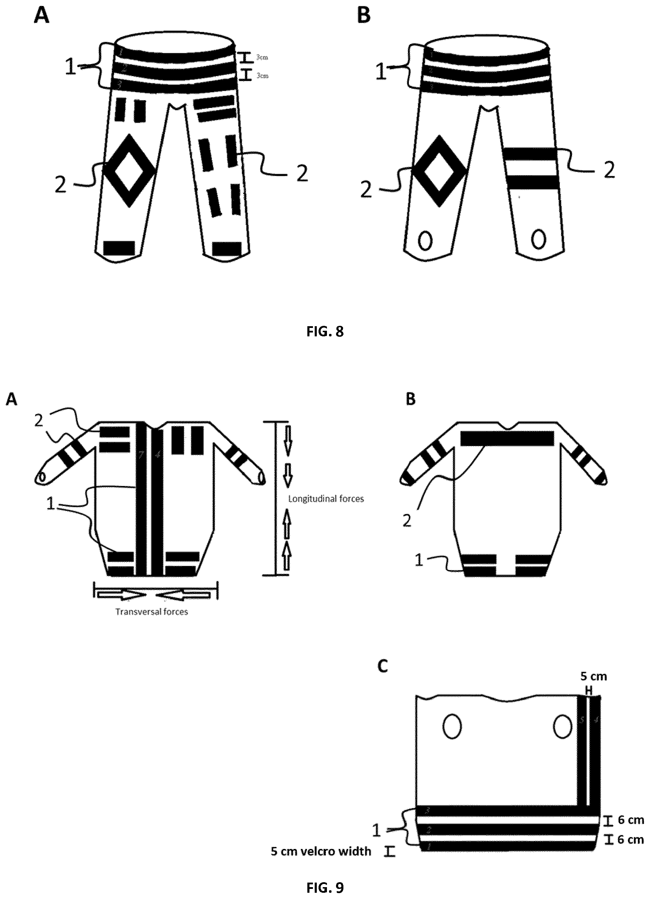

Figure 8 shows a non-limiting example of preferable distribution and position of the fastening elements of first (1) and second (2) type in the trouser-like module, front view (A), back view (B). In Figure 8, said elements are positioned asymmetrically on the right and left leg to show possible different combinations of said elements. If not required by specific needs of the subject wearing the modular garment system, the fastening elements are preferably positioned symmetrically on the right and left leg.

Figure 9 shows a non-limiting example of preferable distribution and position of the fastening elements of first (1) and second (2) type in the jacket-like module, front view (A), back view (B) and internal view (C). In Figure 9(A) and (B), the fastening elements of second (2) type are positioned asymmetrically on the right and left side to show possible different combinations of said elements. If not required by specific needs of the subject wearing the modular garment system, the fastening elements are preferably positioned symmetrically on the left and right side of the upper body.

Figure 10 shows static and isobaric static analyses of a standing subject (in static position) without wearing the modular garment system according to the present invention. In the present case, the subject does not wear the jacket-like module and the trouser-like module.

Figure 11 shows static and isobaric static analyses of the same subject standing (in static position) wearing the modular garment system according to the present invention. In the present case, the subject wears the jacket-like module and the trouser-like module.

Figure 12 shows dynamic and the isobaric dynamic analyses of the same subject standing (in movement) without wearing the modular garment system according to the present invention. In the present case, the subject does not wear the jacket-like module and the trouser-like module.

Figure 13 shows dynamic and the isobaric dynamic analyses of the same subject (in movement) wearing the modular garment system according to the present invention. In the present case, the subject wears the jacket-like module and the trouser-like module.

Figure 14 shows the front (A) and back (B) view of the jacket-like module comprising fastening elements of first (1) and second (2) type according to the present invention. Figure 14(A) shows circumference of the waist of 105 cm, length from below the neck to the waist of 57,5 cm and length from the back part of the neck to the waist of 66,7 cm. Figure 14(B) shows upper back circumference of 115 cm, length from the back part of the neck to the waist of 66,7 cm and length from the middle part of the back part of the neck to the waist of 63,5 cm.

Figure 15 shows back (A) and front (B) view of the trouser-like module comprising fastening elements of first (1) and second (2) type according to the present invention. Figure 15(A) shows circumference of the waist of 83 cm and length from the waist to the ankle of 96,5 cm.

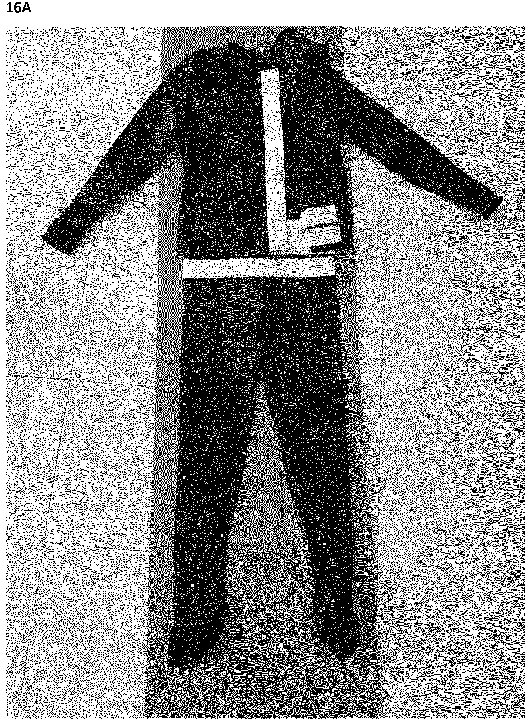

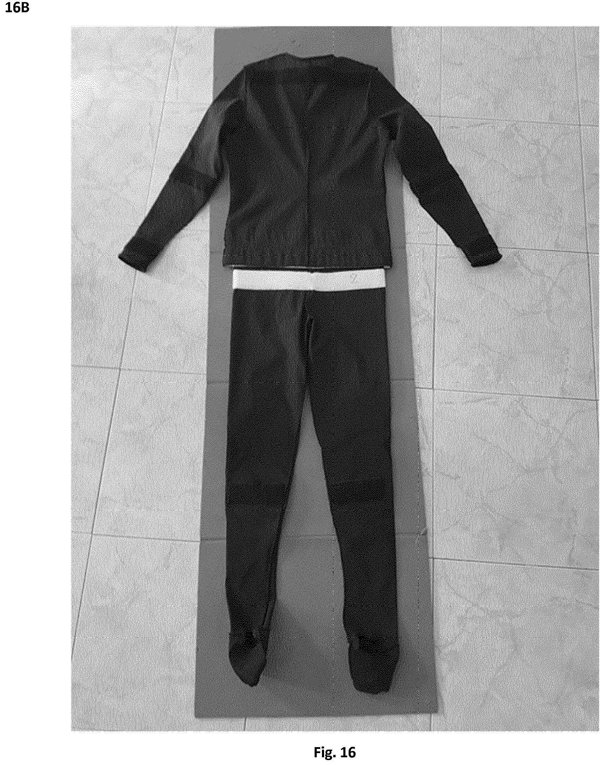

Figure 16 shows front (A) and back (B) view of the modular garment system comprising fastening elements of first (1) and second (2) type according to the present invention.

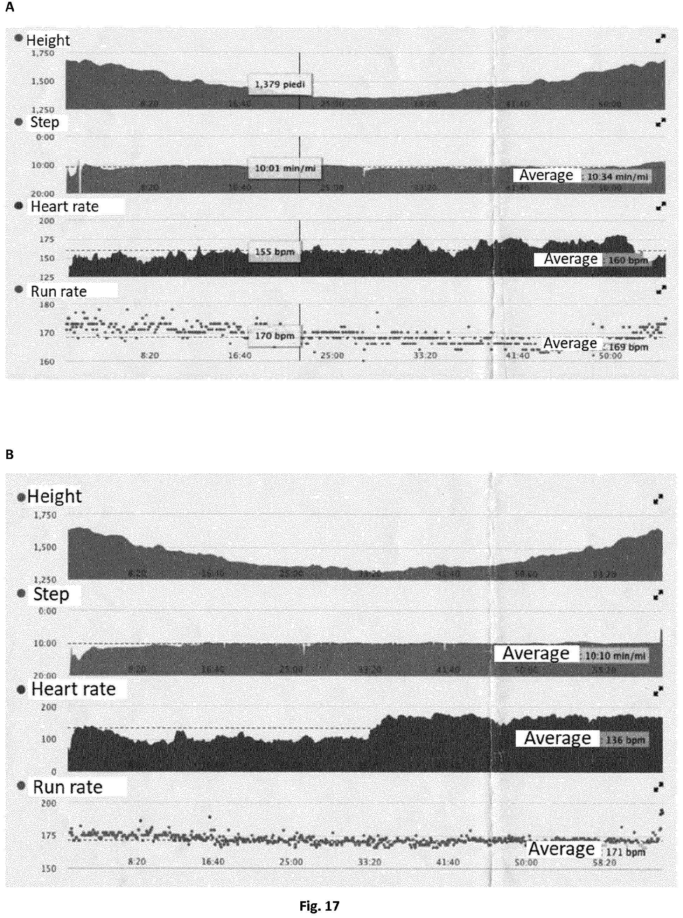

Figure 17 shows values regarding step, heart rate and run rate of a subject while running. Figure 17(A) shows values regarding step, heart rate and run rate of a subject while running without the modular garment system according to the present invention. Figure 17(B) shows values regarding step, heart rate and run rate of a subject while running with the modular garment system according to the present invention.

## DETAILED DESCRIPTION OF THE INVENTION

Disclosed herein is a modular garment system intended to provide enhanced physical performance and efficient rehabilitation in a subject wearing at least one module of such modular garment system. The modular garment system of the present invention offers a unique combination of features as evidenced hereinafter in different aspects and embodiments.

A first advantage provided by the garment system of the present invention lies in its modularity, which can be translated in providing a highly-customized solution to a subject in need thereof.

According to a first aspect of the present invention, there is provided a modular garment system, the system comprising:

a. a jacket-like module, and
b. a trouser-like module

wherein each of said modules comprises at least two fastening elements configured to provide a resistance and/or a support to the movement of a subject's body being contacted by said at least two fastening elements and a compression to said subject's body being contacted by said at least two fastening elements.

In the context of the present invention, the use of a single module of the modular garment system can be sufficient for obtaining improved physical performance and/or rehabilitative results in a subject wearing said single module. Ideally, the use of two modules of said modular garment system provides a superior effect in a subject wearing said modules when performing physical and/or rehabilitative activities.

As used herein, the term "modular" refers to the provision of removable and/or attachable single module (unit) in a garment system, which may be removed and/or applied according to the subject's needs. The term "modular" also refers to the possibility of adapting, modifying and/or modulating the forces exerted by the individual modules and/or the complete modular system (i.e., the system composed by the jacket-like, trouser-like modules, and, optionally, with a hood-like module), which are controlled by means of the characteristics of the fabric and structural elements, by means of the fastening elements of first and second type, based on the specific needs required by the subject wearing the modular garment system disclosed herein. In the context of the present invention, the term "jacket-like module" refers to any garment that is structurally conformed to cover at least one region of the subject's body above the waist and below the neck. Therefore, the "jacket-like module" can comprise garments such as a jacket, a sleeveless jacket, e.g. a gilet, a shoulder sleeve, a sleeve or a combination thereof. In case of the use of the jacket-like module alone, this module can be produced with a sort of pant, short or inguinal support, which would act as a distal anchorage point. Additional anchorage points lie at the neck and at the hands level (for example, thumb hole, finger glove, and/or any method that binds the distal point of the jacket sleeve to the hand/wrist, so as to prevent the rise of the fabric). This arrangement is intended to maintain the modules in the correct position and to ensure that these modules can exert and modulate the required forces, to be able to carry out any movement without loss of tension. Similarly, the term "trouser-like module" refers to any garment that is structurally conformed to cover at least one region of the subject's body below the waist and above the ankle. Therefore, the "trouser-like module" can comprise garments such as tights, leggings and shorts. The trouser-like module, if used alone, can also be produced with shoulder straps and a hole that allows distal anchoring of the heel. These are intended as proximal and distal anchorage points to ensure that these modules exert the required forces. The pant, the shorts, the inguinal support for the jacket-like module, and the shoulder straps for the trouser-like module can be produced as extension of the individual modules or they could be joined to the modules through the fasteners of the first type (1), being removable. The inguinal support and the shoulder straps are not necessary when using and wearing the complete modular garment system since jacket-like and trouser-like module are joined by the fastening elements of first type (1). The modular garment system according to the present invention optionally comprises a hood-like module. The presence of said hood-like module is required when the muscles of head and neck of a subject necessitate to be solicited. In the context of the present invention, the terms "jacket-like", "trouser-like" and "hood-like" refer to wearable garment products, tightly worn to a subject's body, preferably adjacently to the skin. As used herein, the term "subject" refers to a human subject. Said subjects can be healthy subjects or subjects affected by postural and/or muscle disorders. The garment system can also be produced in a single module (e.g., a jumpsuit, with a Velcro placed longitudinally from the pubis area to the neck to allow easy dressing). In this configuration, the forces can be modulated by special fastening elements that can be anchored to different fastening elements of the first type (1) and second type (2).

The modular garment system according to the present invention comprises a preferred combination of modules that are essential for achieving the desired results in terms of improved physical performance and/or efficient recovery in a subject wearing such modular garment system. In the context of the present invention, it is preferred to combine at least two modules of the modular garment system to obtain an improved effect in the subject wearing said at least two modules.

Each module can be actively connected with an adjacent module by means of fastening elements. Each module is characterized by the presence of at least two fastening elements.

According to one embodiment of the first aspect of the present invention, said at least two fastening elements comprise

a. at least one fastening element of first type (1), and
b. at least one fastening elements of second type (2),

wherein said at least one fastening elements of first type (1) is arranged in an overlapping region between two adjacent modules and/or in an overlapping region within the same module, and wherein said at least one fastening element of second type (2) is arranged in a region of the module contacting a skeletal muscle of a subject's body.

In one embodiment, the fastening elements of second type (2) are positioned in region of the subject's body wherein they mimic proximal and distal attachments of the muscles and/or muscular chains and/or fascial chains. The position and configuration of the fastening elements of second type (2) also depends on the subject's specific requirement.

The modular garment system according to the first aspect of the present invention is capable of generating a system of compression forces on the subject's body covered by at least one module. These compression forces are generated by opportunely adhering said fastening elements of first type (1) to each other. As matter of example, the jacket-like module may comprise two fastening elements of first type (1) arranged to adhere to each other and therefore, determining the closure of the jacket-like module on the body of a subject. Preferably, said two fastening elements of first type (1) are arranged in a frontal position of said jacket-like module. The jacket-like module can further comprise one or more additional fastening elements of first type (1) arranged in a lower portion of said jacket-like module and intended to adhere to the upper portion of the trouser-like module, when it is required its presence in the modular garment system, wherein said upper portion of the trouser-like module may comprise one or more fastening elements of first type (1).

In the context of the present invention, a first type of compression and/or resistance and/or tension force on the subject's body is generated by wearing at least one of the module of the modular garment system according to the present invention. A further compression and/or resistance and/or tension force can be generated by combining fastening elements of first (1) type. An additional compression and/or resistance and/or tension force can be generated by combining fastening elements of first (1) and second (2) type comprised in each module of the modular garment system disclosed herein with an elastic band (3).

According to another embodiment of the first aspect of the present invention, the modular garment system may further comprise an elastic band (3). Said elastic band (3) may comprise one or more fastening elements of second type (2) that are intended to adhere to the fastening elements of first (1) and/or second type (2) comprised in the jacket-like module and/or trouser-like module, if required, for generating a system of tension forces on the body of the subject covered by said elastic band (3).

According to another embodiment of the first aspect of the present invention, said elastic band (3) comprises at least one region arranged to contact said at least one fastening element of first (1) and/or second type (2).

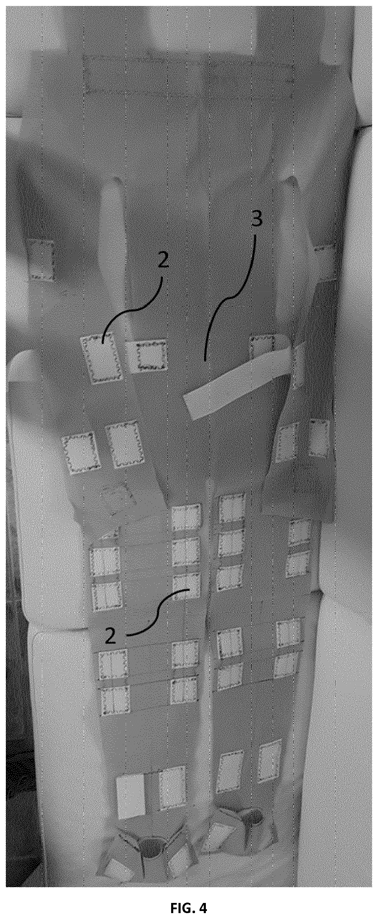

According to another embodiment of the first aspect of the present invention, said elastic band (3) is made by any elastic fabric comprising, but not limited to, elastic Velcro, Elastam or a combination thereof. In a particular embodiment, said elastic band (3) is made by a composition of elastic fabric comprising Velcro and Elastam, wherein the percentage of Velcro ranges from 5 to 50%, preferably from 15 to 45%, more preferably from 20 to 40%. Ideally, the percentage of Velcro corresponds to 30% of the composition. The elastic band depicted in Figure 4 represents a non-limiting particular example. However, in the context of the present invention, suitable elastic band can be exemplified as a single elastic band element that connects one or more fastening element of first (1) and/or principally of second type (2) positioned in the same module and/or in a second module, e.g. the jacket-like module and/or trouser-like module.

The fastening elements of first (1) and second (2) types can be stripes, buttons, clips or other elements which allow the module to preserve its elastic properties to the maximum. In particular, the fastening elements of first type (1) provide a solid anchorage point as they are intended to connect the jacket-like module with the trousers-like module and therefore also define the desired compression/tension/resistance force to be exerted on the subject when said subject is in anatomical position. Differently, the fastening elements of second (2) type are intended to increase the compression/tension/resistance force both at the global level and/or in specific body's segments, thus allowing to oppose a resistance or offer a facilitation to the movement.

According to another embodiment, the area covered by the fastening element of first type (1) and/or second type (2) on each module of the modular garment system of the present invention ranges from 5 to 60%, preferably from 10 to 50%, more preferably from 30 to 40%. The inventor has found that in order to achieve the proper compression/tension/resistance forces on the subject's body wearing the modular garment system, the area covered by the fastening element of first type (1) and/or second type (2) on each module should not exceed 50% to avoid or minimize generating area in each module wherein the elasticity is reduced.

As used herein, the term "compression" refers to a force or system of forces that the fabric of the modular garment system applies to different body's area of a subject wearing said system. In particular, said "compression" is applied to the body's area of a subject predominantly in transverse direction and to a lesser extent in longitudinal direction. This distribution of forces provides beneficial effects to the lymphatic and venous systems of a subject wearing the modular garment system according to the invention.

As used herein the term "longitudinal" refers to the imaginary anatomical plane which divides the body into right and left parts (vertical axis). As used herein, the term "transversal" refers to the imaginary anatomical plane that divides the body into superior and inferior parts (horizontal axis). Therefore, when the terms "longitudinal" and "transversal" are used to define a direction, said direction is oriented according to the corresponding axis.

As used herein, the term "tension" or "resistance" refers to a force or system of forces that the fabric of the modular garment system applies to different body's area of a subject wearing said system. Said force or system of forces is also intended to provide a support to the movement. In particular, said "tension" or "resistance" is applied to the body's area predominantly in longitudinal direction and to a lesser extent in transverse direction. This distribution of forces creates a tension or resistance to the subject's movement, which in a long-term period determines an improvement of the muscle strength, tone and trophism of the resistance in a subject wearing the system according to the invention. An increment of the tension provides with a resistance to the movement of a subject wearing the module garment system, it also offers support to movement according to the subject's needs.

The combination of compression forces with tension/resistance forces provides a stabilizing effect to the articular system of the subject wearing the modular garment system disclosed herein.

In the context of the present invention, the inventor has found that when two modules of the present modular garment system are combined by contacting fastening elements of first (1) and second (2) type with an elastic band (3), the body's area of the subject covered by said modules is subjected to optimal compression and tension forces. In particular, fastening elements of first type (1) from a first module, e.g. the jacket-like module, can be connected with fastening elements of first type (1) from the same module (jacket vertical anchoring) and/or from a second module, e.g. the trouser-like module. Analogously, fastening elements of second type (2) from the same module and/or from a second module, e.g. the trouser-like module. Notably, while fastening elements of first type (1) from the same and/or different modules can be directly connected each other, fastening elements of second type (2) from the same and/or different modules are indirectly connected by means of the elastic band (3).

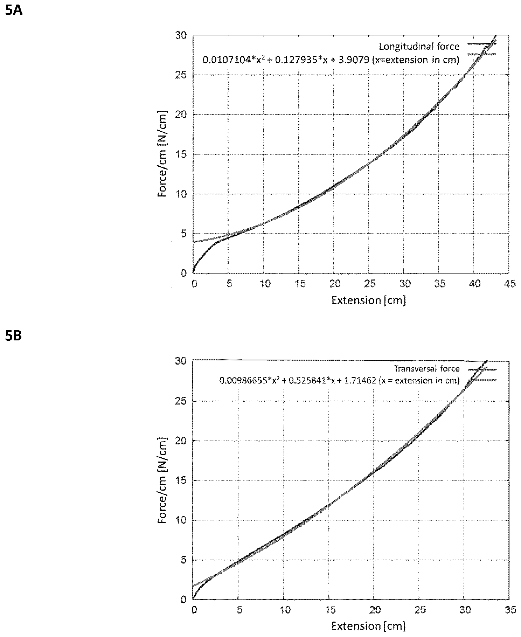

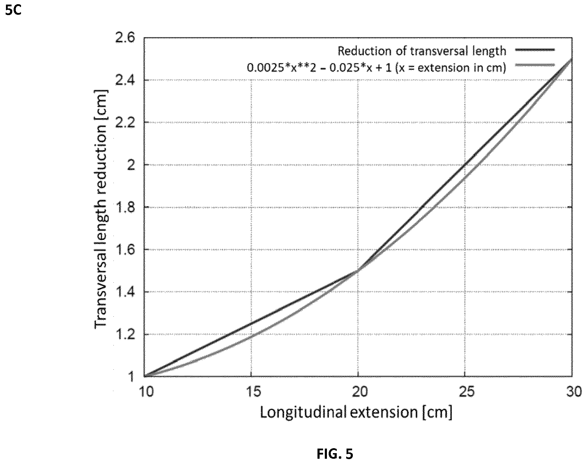

The inventor has found that a longitudinal elongation in the range 0-10 cm induces a longitudinal force per unit of transverse length in the range 0-15 N/cm to be multiplied by the total transverse length considered. Similarly, a longitudinal elongation in the range 10-20 cm induces a longitudinal force per unit of transverse length in the range 15-30 N/cm to be multiplied by the total transverse length considered. Similarly, a longitudinal elongation in the range 20-30 cm induces a longitudinal force per unit of transverse length in the range 30- 45 N/cm to be multiplied by the total transverse length considered. Similarly, a longitudinal elongation in the range 30-40 cm induces a longitudinal force per unit of transverse length in the range 45-65 N/cm to be multiplied by the total transverse length considered. Similarly, a longitudinal elongation in the range 40-50 cm induces a longitudinal force per unit of transverse length in the range 65-100 N/cm to be multiplied by the total transverse length considered (see Figures 5 and 6).

According to one embodiment, the fastening elements comprised in a modular garment system of the present invention are made by elastic fabric.

According to another embodiment, the elastic fabric is selected from the group comprising elastic Velcro, Elastam or a combination thereof.

According to another embodiment of the first aspect of the present invention, the elastic fabric is capable of returning to its original shape and length after being stretched.

In addition to the system of compression/tension/resistance forces exerted to the subject's body by the combination of fastening elements of first (1) and second (2) type comprised in each module of the present modular garment system, the composition of different fabrics characterizing a single module may also influence the tensile properties of said module and thus, contribute on the resultant compression/tension exerted by said module on the subject's body. In particular, the amount of each fabric within the composition may affect the resulting compression of a single garment module on the subject's body covered by said module.

The degree of compression is classified by several standards. In general, low compression refers to pressure of less than 20 mm Hg or class 1; medium compression to pressure of 20-30 mm Hg or class 2; high compression to pressure of greater than 30 mm Hg, or class 3 or higher.

The fabric of each garment module should also satisfy specific prerequisite in order to support the stress due to the movement of the subject's body and the compression generated by each module. Therefore, in the context of the present invention said fabric is characterized by several properties comprising elasticity, breathability, lightness, stretchability and for being non-allergenic and nonsensitizing.

In one embodiment, each module of the modular garment system disclosed herein is made by fabrics being elastic, breathable, lightweight, adherent, hypoallergenic and adaptable to a subject's body.

According to another embodiment, each module of the modular garment system of the present invention is removably mounted to said garment system.

In the context of the present invention, this represent an important property since the subject intending to use the present modular garment system may have many options due to the different possible combinations of the garment modules to achieve the desired result.

Despite psychological effects connected to the "garment feeling" might contribute to the physical performance of the subject wearing such garment, the inventor observed that these effects are irrelevant. In particular, in the context of the present invention, it is excluded any placebo effect since the increase of tension or resistance applied to the subject's body is demonstrated by repetitive tests performed in different subjects.

In the context of the present invention, the fastening elements are intended to act on the muscle tone, muscular force, resistance, muscle mass, fascial system and, more in general, on the skeletal muscular system.

Accordingly, the present modular garments system is suitable for being used in subjects affected by postural disorders, such as kyphosis, scoliosis, lordosis and similar disorders. The present modular garment system is also suitable for being used in subjects affected by muscle disorder such as muscle weakness.

Surprisingly, the inventor has found that by using the modular garment system according to the present invention the altered center of pressure (COP) of a subject having postural disorder can be corrected and aligned with his barycenter.

In the context of the present invention, the term "subject" refers to a subject wearing at least one module of the garment system.

In the context of the present invention, the term "parameters" refers to measurable parameters characterizing a physical activity of a subject. Examples of parameters, without limitation, may comprise wall squat jump, standing long jump, long jump with feet together. As used herein, the term "physical performance" refers to a measurable result of any physical activity performed by a subject.

According to a second aspect of the present invention, a kit for rehabilitation and/or training of a subject is claimed, the kit comprising:

> a. a modular garment system according to the first aspect of the present invention,
> b. an elastic band (3), and
> c. optionally, a hood-like module,

wherein the elastic band (3) is attached to the modular garment system by means of fastening elements of second type (2) arranged on said band (3) that contact corresponding fastening elements of first (1) and second (2) type arranged on the jacket-like module and trouser-like module.

## EXAMPLES

*Mechanical properties of the fabric characterizing each module of the modular garment system*

Tensile tests were performed using a Hounsfield HTE dynamometer model with calibration certificate B01401/7. The measurements were performed on different fabric samples measuring 20cm · 10cm each as follows:

- Constant load elongation, tensile to load test [xhead], internal method; a force of 300 N is applied to the fabric in some tests in the longitudinal direction, in others in the transverse direction, and elongation is measured.
- Elasticity, residual modulus test [xhead], internal method; 5 cycles at 10 and 30 kg with release; distance between the terminals 20cm; traction speed of 300 mm/min; measurements after 2 minutes, 24 hours, 48 hours.
- Resistance to perforation, Persoz compression test [xheah] UNI 5421; the dimensions of the sphere used in the test is 2cm.
- Traction test, Grab test, ISO 13934 part 1; sample sizes analyzed 20cm · 10cm, initial distance between clamps 20 cm and 0 N of force.

Each module of the modular garment system, when worn by a subject in standard anatomical position, applies a compression/tension force of 0 N (Newton). Any movement of the subject from this standard anatomical position triggers a system of compression/tension forces, which provide resistance or assistance to the movement. These forces tend to increment as the length of the fabric increases. The precise control of these forces is ensured by the presence of fastening elements of first (1) and second types (2).

*Example 1*

Figure 5(A) shows physical properties of a fabric sample (20cm*10cm of only fabric, only Elastam without Velcro) measured by an electric dynamometer (Hounsfield HTE). In particular, the graph depicted in Figure 5(A) initially exhibits a non-linear trend, which then evolve into a quadratic trend. Therefore, when an extension is applied in longitudinal sense to the fabric sample, this corresponds to an increment of compression/tension forces.

$$\Delta_{xL} = \text{longitudinal extension/elongation} = 40\text{cm}$$

$$F_{Tot}^L = \text{g}\,(\,\Delta_{xL}\,) = 0{,}0107104*(\,\Delta_{xL}\,)^2 + 0{,}127935 * (\,\Delta_{xL}) + 3{,}9079 = 26{,}161 \text{ N/cm}$$

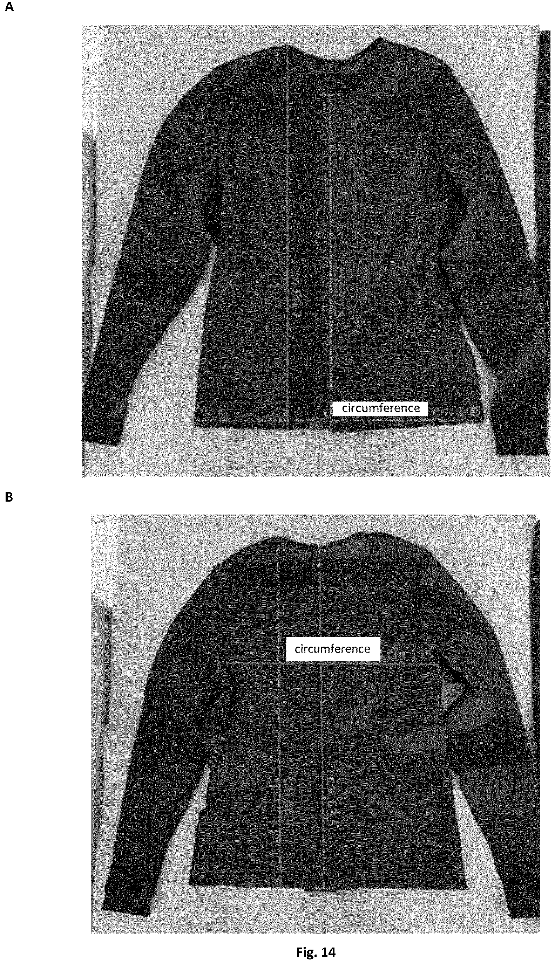

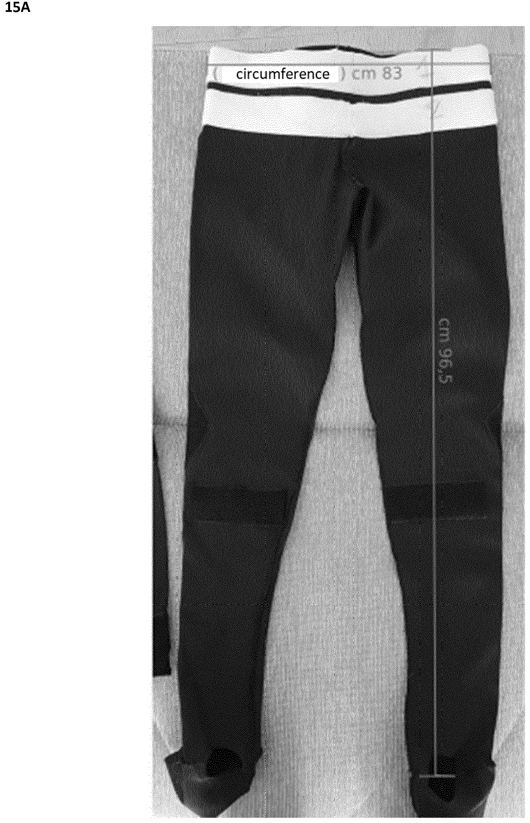

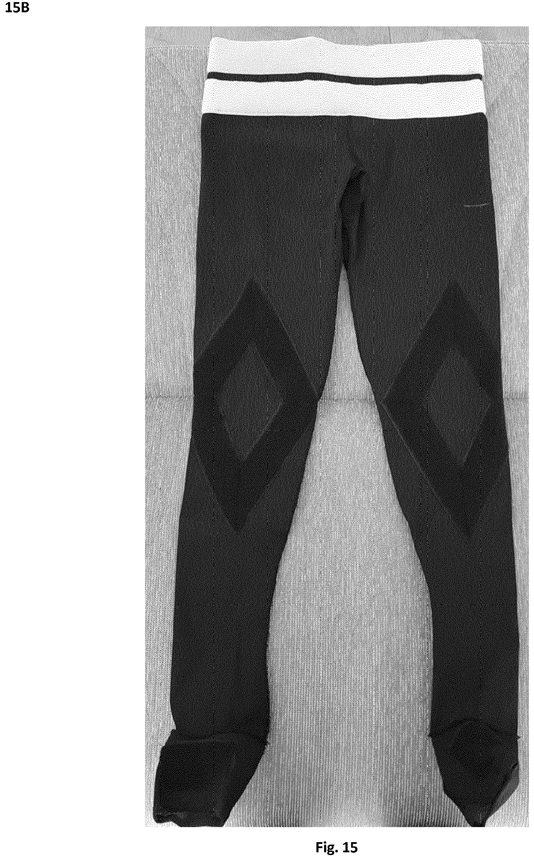

The jacket-like and trouser-like module tested in the present examples are shown in Figures 14 and 15.

A jacket without fastening elements of first (1) second type (2) having a circumference of 95cm, elongated of 40cm and anchored to the fastening elements of first type (1), produces on the subject a longitudinal force of at least 2485,295 N, when the subject wearing the jacket is in standard anatomical position.

26,161 N/cm · 95cm = 2485,295 N

A jacket with the fastening elements of the first (1) type and without fastening elements of the second (2) type and anchored to the fastening elements of the first (1) type, a longitudinal elongation in the range 30/40 cm induces a longitudinal force per unit of transverse length in the range of 45/65 N/cm to be multiplied by the total transverse length considered.

The trend of the graphs depicted in Figures 5(B) and (C) show that an increment of the longitudinal tension corresponds also to an increment of the transversal tension.

Specifically, the Figure 5(B) shows the response of the fabric in terms of force N/cm exerted in a transverse direction following a transversal elongation. It is seen how much transverse force exerts the fabric (without Velcro) following a transversal elongation. Figure 5(C) shows the fabric response in terms of force N/cm exerted in the transverse direction following a longitudinal elongation. The transverse force exerted by the fabric (without Velcro) is seen following a longitudinal extension.

The same jacket with a height of 58cm, closed in frontal position without tension, produce a force of 0 N when the subject is in standard anatomical position. When the jacket is subjected to a longitudinal extension of 40cm, such extension corresponds to an increment of the transverse force of 230.55 N, namely 3.975 N/cm.

Example 1:

$$\Delta_{xL} = \text{longitudinal extension/elongation} = 40\text{cm}$$

$$\Delta_{xt}^0 = \text{initial transverse extension/elongation} = 0\text{cm}$$

$$\Delta_{xt} = \Delta_{xt}^0 + f\!\!\!/\,(\Delta_{xL}) = \text{transverse extension} = 0+4 = 4\text{cm}$$

$$f\!\!\!/\,(\Delta_{xL}) = \text{obtained from equation in graph 3.}$$

$$f\!\!\!/\,(\Delta_{xL}) = 0{,}0025{\cdot}(\Delta_{xL})^2 - 0{,}025 \cdot (\Delta_{xL}) + 1 = 4\text{cm}$$

$$\Delta_{xt} = \Delta_{xt}^0 + f\!\!\!/\,(\Delta_{xL}) = 0 + 4 = 4\text{cm}$$

$$F_{Tot}^T = 0{,}00986655{\cdot}(\Delta_{xt})^2 + 0{,}525841 \cdot (\Delta_{xt}) + 1{.}71462 = 3{,}975 \text{ N/cm}$$

$$3{,}975 \text{ N/cm} \cdot 58\text{cm} = 230{,}55 \text{ N}$$

Example 2:

When the vertical fastening elements of first type (1) of the jacket contact other fastening element of first type (1) of the jacket-like module with a transverse elongation of 5cm and a longitudinal elongation of 40cm, the tension/compression force produced in transversal sense corresponds to

$$\Delta_{xL} = 40\text{cm}$$

$$\Delta_{xt}^0 = 5\text{cm}$$

$$\Delta_{xt} = 5 + 4 = 9$$

$$F_{Tot}^T = 0{,}00986655 \cdot (\Delta_{xt})^2 + 0{,}525841 \cdot (\Delta_{xt}) + 1{,}71462 = 7{,}246 \text{ N/cm}$$

$$7{,}246 \text{ N/cm} \cdot 58\text{cm} = 420{,}268 \text{ N}$$

Example 3:

$$\Delta_{xL} = 10\text{cm}$$

$$\Delta_{xt}^0 = 5\text{cm}$$

$$f(\Delta_{xL}) = 0{,}0025 \cdot (\Delta_{xL})^2 - 0{,}025 \cdot (\Delta_{xL}) + 1 = 1\text{cm}$$

$$\Delta_{xt} = \Delta_{xt}^0 + f(\Delta_{xL}) = 5 + 1 = 6\text{cm}$$

$$F_{Tot}^T = 0{,}00986655 \cdot (\Delta_{xt})^2 + 0{,}525841 \cdot (\Delta_{xt}) + 1{.}71462 = 5{,}224 \text{ N/cm}$$

$$5{,}224 \text{ N/cm} \cdot 58\text{cm} = 302{,}992 \text{ N}$$

Figure 6(C) shows that the forces analyzed are those of a trouser segment comprising a Velcro placed proximally and transversely. Velcro 30% Elastam 70%, it is analyzed the longitudinal force exerted by the fabric (with 30% Velcro transversely positioned) following a longitudinal extension.

Es1:

$$\Delta_{xL} = 40\text{cm}$$

$$F_{Tot}^{Lp} = 0{,}0268257 * (\Delta_{xL})^2 + 0{,}625781 * (\Delta_{xL}) + 1{,}16339 = 69{,}115 \text{ N/cm}$$

$$69{,}115 \text{ N/cm} * 83\text{cm} = 5736{,}545 \text{ N}$$

*Elasticity test of the fabric characterizing each module of the modular garment system of the invention*

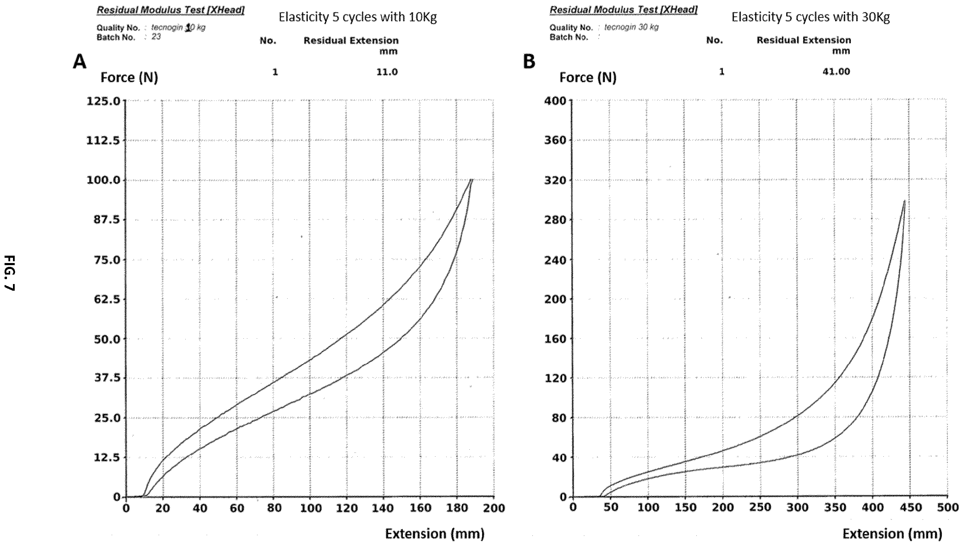

Figure 7(A-B) shows the fabric response to stress. In particular, the tested fabric sample (size, 20 * 10cm) has been stressed up to 100 N (Figure 7A) and up to 300 N (Figure 7B), both for 5 cycles.

In the first case, from the elasticity test carried out for 5 cycles at 100 N, after 2 minutes from the test, the length of the fabric sample measures 20.7cm, having a 3.5% deformity and a recovery of 96.5%. After 24h, the fabric sample measures 20.3cm and after 48h said fabric sample measures 20.1cm (Figure 7A).

In the second case, from the elasticity test carried out for 5 cycles at 300 N, after 2 minutes from the test, the length of the fabric sample measures 21.5cm, having a deformity of 7.5% and a recovery of 92.5%. After 24h, the fabric sample measures 20.7cm and after 48h measures 20.5cm (Figure 7B).

Taking into account that these tests were performed on small tissue samples (20 * 10cm), and having had excellent results, when using larger tissue segments, the deformity is smaller and the recovery greater. Therefore, the adaptation of the individual to the use of the suit, to the forces opposed by this, are not given by the adaptation of the modular system (perhaps with a loss of elasticity from the fabric), but from adaptations, improvements in the body of the individual.

*Physical tests of a subject wearing the modular garment system of the invention*

The modular garment system according to the present invention has been tested on a group of subjects, regular sportsmen, having no medical conditions that would affect the experimental results. As matter of example, the following tests have been performed on a 31-years old subject.

The following tests have been performed in the time frame of one month, organized in 12 workout sessions of 60 minutes each. Before each workout session, the subject performed a warm up session comprising the following exercises: walking preparatory for the run (skipping, butt-kicks, heel-toe walking), running (repeatedly on 10, 20 and 30 meters), barbell and dumbbell workouts.

Importantly, the workout sessions have been set up without focusing on a specific body area to further appreciate the results obtained by the subject wearing the modular garment system according to the present invention. The loading phase does not exceed 30 minutes.

At the end of each training session, the subject performed stretching based on Meziere's postures, one minute for each posture, for 5-6 minutes; the recovery phase lasts 10-20 seconds among the same exercises and 1-2 minutes among the different series.

Table 1 shows the results in terms of improved performance of a subject wearing the jacket-like module and the trouser-like module of the modular garment system according to the present invention.

Table 1. Performance of a subject without wearing the garment system of the present invention (before) vs the same subject wearing the garment system of the present invention (after) measured in terms of explosive power and reactive strength tests.

| Explosive power tests performed: | | Before | After |
|---|---|---|---|
| Wall squat jump | Start (cm) | 215 | 215 |
| | Arrival (cm) | 252 | 270 |
| | Difference (cm) | 37 | 55 |
| Standing long jump | 1° Jump (cm) | 213 | 230 |
| | 2° Jump (cm) | 215 | 230 |
| | 3° Jump (cm) | 219 | 230 |
| | Average (cm) | 215,667 | 230,000 |

(continued)

| Reactive Strength Index (RSI) tests performed: | | Before | After |
|---|---|---|---|
| Long jump with feet together (10 meters) | Bipodalic position (meters) / No. of jumps | 2 / 5 | 3,333 / 3 |
| | Monopodalic position (dx) (meters) / No. of jumps | 1,667 / 6 | 2,000 / 5 |
| | Monopodalic position (sx) (meters) / No. of jumps | 1,667 / 6 | 2,000 / 5 |

*Postural tests*

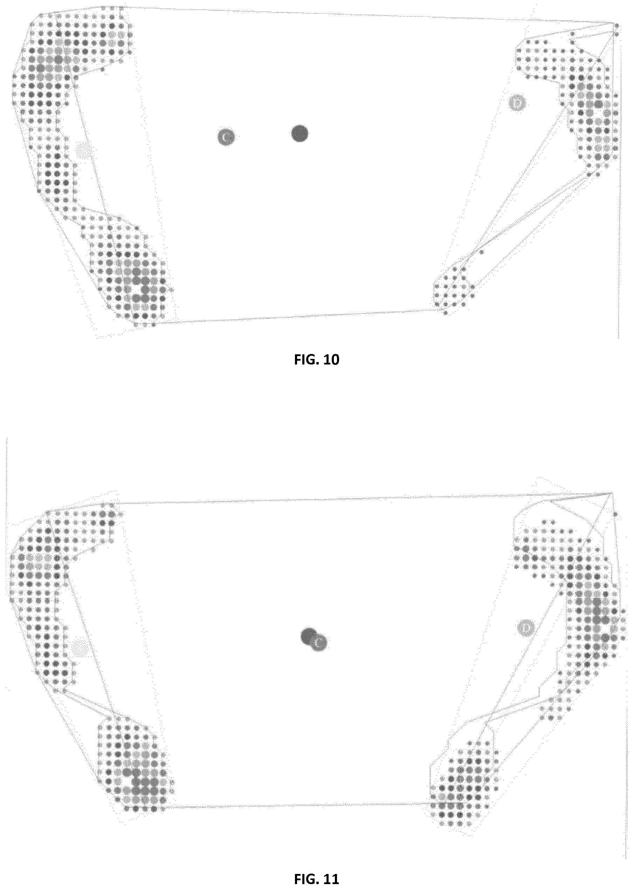

Static and isobaric static analyses of a standing subject (in static position) without wearing the modular garment system according to the present invention are shown in Figure 10. Center of pressure (COP), indicated by the letter C, corresponding to the projection onto the supporting base of the body center of gravity, shows a moderate unilateral overload indicating an unbalanced pressure load on the heel. The center of foot pressure (COF) in each lower limb indicated by the letters S (left) and D (right) shows a misaligned position over the COP. Such non-physiological condition involves rotating attitudes: left limb COF is placed before the COP and right limb COF placed after the COP. Pressure peak (P. Max = High-Pressure Point, indicated by the letter M) has been detected on the right forefoot area, which may indicate the local overload on a single lower limb in presence of other high-pressure points). The load percentage on the single lower limb shows a moderate overload on the left lower limb. The load distribution between the forefoot and the backfoot of the left and right lower limbs is within the physiological values. The surfaces of the two feet were revealed to be non-uniform with an unbalanced load distribution on the left foot. An evident surface difference can be appreciated between the two forefeet, which is greater for the left foot. An evident surface difference can also be appreciated between the two back feet, which is greater for the left foot. Maximum loading points (grey) are not detected on the right foot. Overload area are detected on both forefoot. Average loading points (light grey) are not detected on right back feet.

Static and isobaric static analyses of the same standing subject (in static position) wearing the modular garment system according to the present invention are shown in Figure 11. Center of pressure (COP), indicated by the letter C, corresponding to the projection onto the supporting base of the body center of gravity, shows a moderate unilateral overload indicating an unbalanced pressure load on the heel). The center of foot pressure (COF) in each lower limb indicated by the letters S (left) and D (right) shows a misaligned position over the COP. Such non-physiological condition involves rotating attitudes The COF is located back for the left lower limb and forward for the right lower limb. Pressure peak (P. Max = High-Pressure Point, indicated by the letter M) has been detected on the right forefoot area, which may indicate the local overload on a single lower limb in presence of other high-pressure points). The load percentage on each lower limb shows an excessive overload on the right lower limb. The load distribution between the forefoot and the backfoot of the left and right lower limb is within the physiological values. The surface of the two feet were measured with similar distribution. A slight surface difference can be appreciated between the two forefeet, which is greater for the right foot. A slight surface difference can also be appreciated between the two back feet, which is greater for the left foot. Maximum loading points (grey) are not detected on the right foot. Overload area are detected on the right forefoot. Average loading points (light grey) are detected on both back feet, but more evident on the left foot.

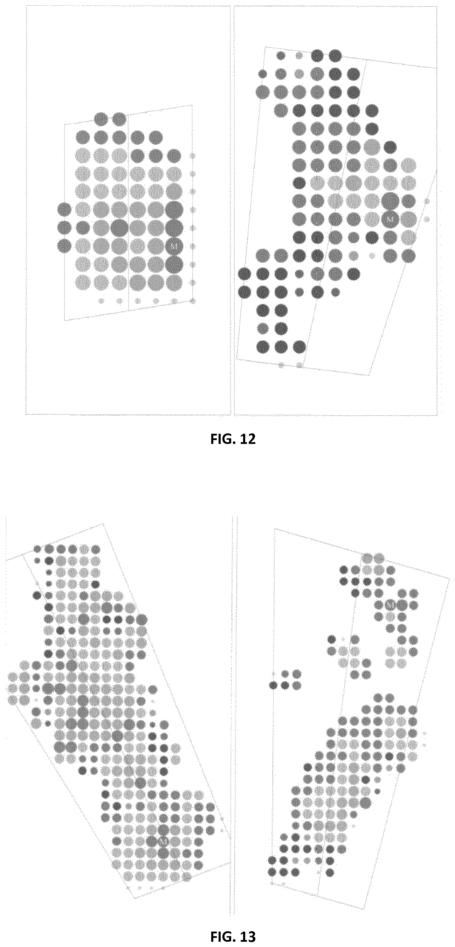

Dynamic and isobaric dynamic analyses of the same standing subject (in movement) without wearing the modular garment system according to the present invention are shown in Figure 12. Pressure peak (P. Max = High-Pressure Point, indicated by the letter M) has been detected on the right forefoot area. The load percentage on each lower limb shows a moderate overload on the right lower limb. The load distribution between the forefoot and the backfoot of the left and right lower limb is within the physiological values. A non-uniform surface pressure of the two feet have been found, being greater for the right foot. Between the two front feet can be also appreciated an excessive surface difference, being greater for the right front foot. Maximum loading points (grey) are not detected on the right foot, while they are slight detected on the left one. Average loading points (light grey) are not detected on the back of right foot.

Dynamic and the isobaric dynamic analyses of the same standing subject (in movement) wearing the modular garment system according to the present invention are shown in Figure 13. Pressure peak (P. Max = High-Pressure Point, indicated by the letter M) has been detected on the right forefoot area. The load percentage on each lower limb shows a moderate overload on the left lower limb. The load distribution between the forefoot and the backfoot of the left and right lower limb is within the physiological values. A non-uniform surface pressure of the two feet have been found, being greater for the left feet. Between the two forefeet can be appreciate a noticeable surface difference, being greater for the left lower limb. Between the two back feet can be also appreciated an excessive surface difference, being greater for the left lower limb. Maximum loading points (grey) are detectable on both feet, but they are predominant for the left one. Average loading points (light grey) are detected on the back of both feet, but mostly on the left foot.

The use of the modular garment system according to the present invention provides beneficial effect in a subject affected by postural disorders as evidenced in Figures 11 and 13. The system of compression and resistance/tension forces exerted on the body of a subject wearing the present modular garment system attempts to keep different body segments closer to the body's longitudinal axis either when the subject is in anatomical position or when the subject performs movements altering said anatomical position.

Therefore, by controlling the execution of the subject's movement and, more in general, by imposing the adoption of an ideal posture position in said subject, the present modular garment system provides a stabilizing effect along different body sectors in said subject.

A subject affected by postural disorders shows a misalignment of the COP with his barycenter (Figure 10). Figures 11 shows that the COP of a subject wearing the modular garment system disclosed herein is almost aligned with his barycenter. The stabilizing effect can be also appreciated in terms of better load distribution on lower limbs in a subject wearing the present modular garment system (see Figures 12 and 13).

**Claims**

1. A modular garment system, the system comprising:

   a. a jacket-like module, and
   b. a trouser-like module,
   wherein each of said modules comprises at least two fastening elements configured to provide a resistance to the movement of a subject's body being contacted by said at least two fastening elements and a compression to said subject's body being contacted by said at least two fastening elements,
   wherein said at least two fastening elements are made by an elastic fabric selected from the group comprising elastic Velcro, Elastam or a combination thereof,
   wherein said at least two fastening elements comprise

   a. at least one fastening element of first type (1), and
   b. at least one fastening element of second type (2),

   wherein said at least one fastening element of first type (1) is arranged in an overlapping region between two adjacent modules and in an overlapping region within the same module, and wherein said at least one fastening element of second type (2) is arranged in a region of the module contacting a skeletal muscle of said subject's body.

2. The modular garment system according to claim 1, the system further comprises an elastic band (3).

3. The modular garment system according to claims 1 and 2, wherein said elastic band (3) comprises at least one region arranged to contact said at least one fastening element of second type (2).

4. The modular garment system according to any of the claims 1 to 3, wherein each of said modules is made by a fabric being elastic, breathable, lightweight, adherent, hypoallergenic and adaptable to a subject's body, wherein the fabric comprises elastic Velcro, Elastam or a combination thereof.

5. The modular garment system according to any of the claims 1 to 4, wherein each of said modules is removably mounted to said modular garment system.

6. A kit for rehabilitation and/or training of a subject, the kit comprising:

   a. a modular garment system according to any of the claims 1 to 5
   b. an elastic band (3), and
   c. optionally, a hood-like module,

   wherein the elastic band (3) is attached to the modular garment system by means of fastening elements of second type (2) arranged on said band (3) that contact corresponding fastening elements of first (1) and second (2) type arranged on the jacket-like module and trouser-like module.

**Patentansprüche**

1. Ein modulares Bekleidungssystem, wobei das System umfasst:

   a. ein jackenartiges Modul, und
   b. ein hosenartiges Modul,
   wobei jedes der Module mindestens zwei Befestigungselemente umfasst, die so konfiguriert sind, dass sie der Bewegung des Körpers einer Person, der von den mindestens zwei Befestigungselementen berührt wird, einen Widerstand entgegensetzen und auf den Körper der Person, der von den mindestens zwei Befestigungselementen berührt wird, eine Kompression ausüben,
   wobei die mindestens zwei Befestigungselemente aus einem elastischen Stoff bestehen, der aus der Gruppe ausgewählt ist, die elastischen Klettverschluss, Elastam oder eine Kombination davon umfasst,
   wobei die mindestens zwei Befestigungselemente Folgendes umfassen

   a. mindestens ein Befestigungselement des ersten Typs (1), und
   b. mindestens ein Befestigungselement des zweiten Typs (2),

   wobei das mindestens eine Befestigungselement des ersten Typs (1) in einem überlappenden Bereich zwischen zwei benachbarten Modulen und in einem überlappenden Bereich innerhalb desselben Moduls angeordnet ist, und
   wobei das mindestens eine Befestigungselement des zweiten Typs (2) in einem Bereich des Moduls angeordnet ist, der einen Skelettmuskel des Körpers der Person berührt.

2. Das modulare Bekleidungssystem nach Anspruch 1, wobei das System ferner ein elastisches Band (3) umfasst.

3. Ein modulares Bekleidungssystem nach den Ansprüchen 1 und 2, wobei das elastische Band (3) mindestens einen Bereich umfasst, der so angeordnet ist, dass er das mindestens eine Befestigungselement des zweiten Typs (2) berührt.

4. Das modulare Bekleidungssystem nach einem der Ansprüche 1 bis 3, wobei jedes der Module aus einem Stoff besteht, der elastisch, atmungsaktiv, leicht, haftend, hypoallergen und an den Körper einer Person anpassbar ist, wobei der Stoff, elastischen Klettverschluss, Elastam oder eine Kombination davon umfasst.

5. Das modulare Bekleidungssystem nach einem der Ansprüche 1 bis 4, wobei jedes der Module abnehmbar an dem modularen Bekleidungssystem angebracht ist.

6. Ein Bausatz für die Rehabilitation und/oder das Training einer Person, wobei der Bausatz Folgendes umfasst:

   a. ein modulares Bekleidungssystem nach einem der Ansprüche 1 bis 5
   b. ein elastisches Band (3), und
   c. optional, ein kapuzenartiges Modul,

   wobei das elastische Band (3) an dem modularen Bekleidungssystem mittels Befestigungselementen des zweiten Typs (2) befestigt ist, die an dem Band (3) angeordnet sind und entsprechende Befestigungselemente des ersten (1) und zweiten (2) Typs berühren, die an dem jackenartigen Modul und dem hosenartigen Modul angeordnet sind.

**Revendications**

1. Système de vêtement modulaire, le système comprenant:

   a. un module en forme de veste, et
   b. un module en forme de pantalon,
   dans lequel chacun desdits modules comprend au moins deux éléments de fixation configurés pour fournir une résistance au mouvement du corps d'un sujet étant en contact avec lesdits au moins deux éléments de fixation et une compression audit corps du sujet étant en contact avec lesdits au moins deux éléments de fixation,
   dans lequel lesdits au moins deux éléments de fixation sont constitués d'un tissu élastique choisi dans le groupe comprenant le Velcro élastique, l'Elastam ou une combinaison de ceux-ci,

dans lequel lesdits au moins deux éléments de fixation comprennent

    a. un. au moins un élément de fixation du premier type (1), et
    b. au moins un élément de fixation du deuxième type (2),

dans lequel ledit au moins un élément de fixation du premier type (1) est disposé dans une région de chevauchement entre deux modules adjacents et dans une région de chevauchement à l'intérieur du même module, et dans lequel ledit au moins un élément de fixation du deuxième type (2) est disposé dans un région du module en contact avec un muscle squelettique du corps dudit sujet.

2. Système de vêtement modulaire selon la revendication 1, le système comprend en outre une bande élastique (3).

3. Système de vêtement modulaire selon les revendications 1 et 2, dans lequel ladite bande élastique (3) comprend au moins une région agencée pour entrer en contact avec ledit au moins un élément de fixation du deuxième type (2).

4. Système de vêtement modulaire selon l'une quelconque des revendications 1 à 3, dans lequel chacun desdits modules est constitué d'un tissu élastique, respirant, léger, adhérent, hypoallergénique et adaptable au corps d'un sujet, dans lequel le tissu comprend du Velcro élastique, de l'élasthanne ou une combinaison de ceux-ci.

5. Système de vêtement modulaire selon l'une quelconque des revendications 1 à 4, dans lequel chacun desdits modules est monté de manière amovible sur ledit système de vêtement modulaire.

6. Kit de rééducation et/ou d'entraînement d'un sujet, le kit comprenant :

    a. un système de vêtement modulaire selon l'une quelconque des revendications 1 à 5
    b. une bande élastique (3), et
    c. éventuellement, un module de type capuche,

dans lequel la bande élastique (3) est fixée au système de vêtement modulaire au moyen d'éléments de fixation du deuxième type (2) disposés sur ladite bande (3) qui entrent en contact avec les éléments de fixation correspondants du premier (1) et du deuxième (2) types disposés sur le module en forme de veste et le module en forme de pantalon.

**FIGURES**

FIG. 1

A

B

FIG. 2

A                    B

FIG. 3

FIG. 4

**5A**

**5B**

5C

FIG. 5

**6A**

**6B**

6C

FIG. 6

**Residual Modulus Test [XHead]**

Quality No. : *tecnogin 10 kg*
Batch No. : 23

Elasticity 5 cycles with 10Kg

| No. | Residual Extension mm |
|---|---|
| 1 | 11.0 |

**A**

Force (N)

**Residual Modulus Test [XHead]**

Quality No. : *tecnogin 30 kg*
Batch No. :

Elasticity 5 cycles with 30Kg

| No. | Residual Extension mm |
|---|---|
| 1 | 41.00 |

**B**

Force (N)

FIG. 7

EP 3 713 435 B1

**FIG. 8**

**FIG. 9**

FIG. 10

FIG. 11

FIG. 12

FIG. 13

A

B

Fig. 14

15A

15B

Fig. 15

16A

16B

Fig. 16

**A**

**B**

Fig. 17

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4215687 A
- US 20110009793 A1
- US 20070179421 A1
- CA 2297593
- US 4961418 A

**Non-patent literature cited in the description**

- **BORN et al.** *Int. J. Sports Physiol. Perform.,* 2013, vol. 8 (1), 4-18
- **BRINGARD et al.** *Int. J. Sports Med.,* 2006, vol. 27 (5), 373-378
- **SCANLAN et al.** *Int. J. Sports Physiol. Perform.,* 2008, vol. 3 (4), 424-438